(19) Europäisches Patentamt
European Patent Office
Office européen des brevets

(11) EP 0 673 641 B1

# (12) FASCICULE DE BREVET EUROPEEN

(45) Date de publication et mention de la délivrance du brevet:
**09.10.1996 Bulletin 1996/41**

(51) Int. Cl.$^6$: **A61K 7/13**

(21) Numéro de dépôt: **95400224.2**

(22) Date de dépôt: **02.02.1995**

## (54) Composition de teinture d'oxydation des fibres kératiniques comprenant un dérivé de paraphénylènediamine et un polymère substantif cationique ou amphotère et utilisation

Zusammensetzung zur oxidativen Färbung von Keratinfasern enthaltend ein Paraphenylendiaminderivat und eine haftende kationische oder amphotäre Polymär, und dessen Verwendung

Composition for the oxidative dyeing of keratin fibres, comprising a paraphenylene diamine derivative and a settling cationic of amphoteric polymer, and its use

(84) Etats contractants désignés:
**DE ES FR GB IT**

(30) Priorité: **21.03.1994 FR 9403272**

(43) Date de publication de la demande:
**27.09.1995 Bulletin 1995/39**

(73) Titulaire: **L'OREAL**
**F-75008 Paris (FR)**

(72) Inventeurs:
- **Cotteret, Jean**
  **F-78480 Verneuil S/Seine (FR)**
- **Audousset, Marie-Pascale**
  **F-92600 Asnieres (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 007 537** **EP-A- 0 400 330**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 673 641 B1

Printed by Rank Xerox (UK) Business Services
2.13.8/3.4

## Description

La présente invention concerne une composition de teinture d'oxydation des fibres kératiniques et en particulier des fibres kératiniques humaines, comprenant au moins une paraphénylènediamine particulière substituée en position 2 sur le noyau benzénique, et au moins un polymère substantif cationique ou amphotère. Elle concerne également l'utilisation d'une telle composition dans l'application cosmétique susmentionnée.

Il est connu de teindre les fibres kératiniques et en particulier les cheveux humains, avec des compositions de teinture contenant des précurseurs de colorants d'oxydation, en particulier des ortho- ou para- phénylènediamines, des ortho- ou para- aminophénols, généralement appelés "bases d'oxydation", et des coupleurs encore appelés modificateurs de coloration, plus particulièrement des métaphénylènediamines, des méta-aminophénols et des métadiphénols, qui permettent de modifier et d'enrichir de reflets les colorations "de fond" obtenues par les produits de condensation des bases d'oxydation.

Il est ainsi notamment connu de teindre les cheveux avec des compositions de teinture qui contiennent, à titre de précurseurs de colorants d'oxydation, des paraphénylènediamines du type 1-hydroxyalkyle 2,5-diamino benzène ou encore 1-hydroxyalcoxy 2,5-diamino benzène, associées le cas échéant avec un ou plusieurs coupleurs usuels. De telles compositions sont par exemple décrites dans la demande de brevet WO 80/00214 et le brevet EP-B-0 400 330.

Cependant, il est apparu à la demanderesse que, pour être encore plus satisfaisantes, les teintures ci-dessus devaient être moins sélectives, c'est à dire qu'elles devaient être moins sensibles, sur le plan tinctorial, aux divers degrés de sensibilisation des cheveux à teindre, afin que l'écart de couleur observé sur ces cheveux plus ou moins sensibilisés, soit le plus faible possible, et qu'ainsi la chevelure soit teinte uniformément. Il est par ailleurs apparu important que ces compositions puissent générer des teintures dont la puissance d'une part et la résistance à la fois aux agents atmosphériques (lumière, intempéries), à la transpiration et aux différents traitements cosmétiques que peuvent subir les cheveux (lavages, permanentes, et autres) d'autre part, soient encore améliorées.

Or, après d'importantes recherches menées sur la question, la demanderesse vient maintenant de découvrir qu'il est possible d'obtenir des teintures d'oxydation à base de composés de type 1-hydroxyalkyle ou 1-hydroxyalcoxy 2,5-diamino benzène, qui présentent une sélectivité nettement améliorée par rapport à celles jusqu'ici connues, lorsqu'on associe auxdits composés, un polymère substantif cationique ou amphotère.

La demanderesse a également découvert que les teintures obtenues à l'aide de ces nouvelles compositions sont plus puissantes, tout en restant très résistantes aux agents atmosphériques et aux divers traitements cosmétiques susmentionnés.

Ces découvertes sont à la base de la présente invention.

La présente invention a ainsi pour objet une composition de teinture d'oxydation pour fibres kératiniques, en particulier pour fibres kératiniques humaines, telles que les cheveux, du type comprenant, dans un milieu approprié pour la teinture, au moins un précurseur de colorant d'oxydation (base d'oxydation) et, le cas échéant, un ou plusieurs coupleurs, qui est caractérisée par le fait qu'elle contient (i) à titre de précurseur de colorant d'oxydation, au moins une paraphénylènediamine choisie parmi les composés de formule (I) suivante :

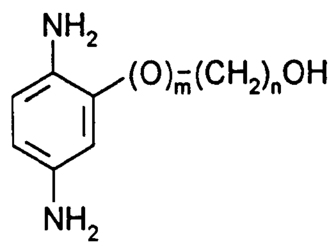

(I)

dans laquelle m est un nombre entier égal à zéro ou 1, et n est un nombre entier compris inclusivement entre 1 et 4, et les sels d'addition de ces composés de formule (I) avec un acide,
et (ii) au moins un polymère substantif cationique ou amphotère.

De préférence, selon l'invention, les précurseurs de colorant d'oxydation de formule (I) ci-dessus sont mis en oeuvre en association avec un ou plusieurs coupleurs.

Les nouvelles teintures obtenues dans le cadre de la présente invention permettent d'aboutir à des colorations puissantes, tenaces et uniformes de la racine à la pointe des cheveux.

Un autre objet de la présente invention porte sur une composition prête à l'emploi pour la teinture des fibres kératiniques, et qui contient au moins une paraphénylènediamine de formule (I) ci-dessus, au moins un polymère substantif cationique ou amphotère, éventuellement un ou plusieurs coupleurs, et au moins un agent oxydant.

L'invention vise également un procédé de teinture des fibres kératiniques, et en particulier des fibres kératiniques humaines telles que les cheveux, consistant à appliquer sur ces fibres au moins une composition (A1) contenant, dans un milieu approprié pour la teinture, au moins une paraphénylènediamine de formule (I) ci-dessus à titre de précurseur de colorant d'oxydation, éventuellement un ou plusieurs coupleurs, en association avec au moins un polymère substantif cationique ou amphotère, la couleur étant révélée à pH alcalin, neutre ou acide à l'aide d'un agent oxydant qui est mélangé juste au moment de l'emploi à la composition (A1) ou qui est présent dans une composition (B1) appliquée simultanément ou séquentiellement de façon séparée.

L'invention vise aussi une variante de ce procédé, qui consiste à appliquer sur les fibres au moins une composition (A2) contenant dans un milieu approprié pour la teinture, au moins une paraphénylènediamine de formule (I) ci-dessus, éventuellement un ou plusieurs coupleurs, et ceci en la présence ou l'absence de polymère substantif, la couleur étant révélée à pH alcalin, neutre ou acide à l'aide d'une composition oxydante (B2) qui contient un agent oxydant et au moins un polymère substantif cationique ou amphotère et qui est mélangée juste au moment de l'emploi à la composition (A2) ou qui est appliquée simultanément ou séquentiellement de façon séparée.

L'invention a également pour objet des dispositifs de teinture ou "kits" à plusieurs compartiments, dont le premier compartiment contient au moins une paraphénylènediamine de formule (I) ci-dessus à titre de précurseur de colorant d'oxydation, éventuellement un ou plusieurs coupleurs, et au moins un polymère substantif cationique ou amphotère, et le deuxième compartiment un agent oxydant.

Selon une autre variante, l'invention a également pour objet des dispositifs de teinture ou "kits" à plusieurs compartiments, dont le premier compartiment contient au moins une paraphénylènediamine de formule (I) ci-dessus à titre de précurseur de colorant d'oxydation, éventuellement un ou plusieurs coupleurs, et ceci en la présence ou en l'absence de polymère substantif cationique ou amphotère, et le deuxième compartiment un agent oxydant et au moins un polymère substantif cationique ou amphotère.

Mais d'autres caractéristiques, aspects, objets et avantages de l'invention apparaîtront encore plus clairement à la lecture de la description et des exemples qui suivent.

Les sels d'acide de la paraphénylènediamine de formule (I) qui peuvent être utilisés selon l'invention sont choisis de préférence parmi les chlorhydrates, les sulfates, les bromhydrates et les tartrates.

Parmi les précurseurs de colorant d'oxydation de formule (I) utilisables dans le cadre de la présente invention, on préfère mettre en oeuvre la 2-(β-hydroxyéthyl) paraphénylènediamine et la 2-(β-hydroxyéthyloxy) paraphénylènediamine.

La concentration en ce(s) précurseurs(s) ou leurs sels peut varier entre 0,05 et 10 % en poids environ par rapport au poids total de la composition de teinture appliquée sur les cheveux, et de préférence entre 0,1 et 5 % en poids environ.

Le caractère substantif (c'est à dire l'aptitude au dépôt sur les cheveux) des polymères cationiques ou amphotères utilisés conformément à l'invention est classiquement déterminé au moyen du test décrit par Richard J. Crawford, Journal of the Society of Cosmetic Chemists, 1980, 31 - (5) - pages 273 à 278 (révélation par colorant acide Red 80).

Ces polymères substantifs cationiques ou amphotères peuvent être choisis parmi ceux antérieurement décrits dans la littérature, en particulier dans la demande de brevet EP-A-0 557 203, de la page 4, ligne 19, à la page 12, ligne 14.

On peut citer en outre d'autres dérivés d'éthers de cellulose quaternisés tels que ceux décrits dans la demande EP-A-0 189 935, et en particulier le polymère commercialisé sous la dénomination "Quatrisoft LM 200" par la société Union Carbide; ces polymères sont également définis dans le dictionnaire CTFA (5ème édition, 1993) comme des ammonium quaternaires d'hydroxyéthylcellulose ayant réagi avec un époxyde substitué par un groupement lauryldiméthylammonium et y sont répertoriés sous l'appellation de "Polyquaternium 24".

On peut également citer les produits qui sont dénommés dans le dictionnaire CTFA (5ème édition, 1993) "Polyquaternium 37", "Polyquaternium 32" et "Polyquaternium 35", qui correspondent respectivement, en ce qui concerne le "Polyquaternium 37", au poly(chlorure de méthacryloyloxyéthyltriméthylammonium) réticulé, en dispersion à 50% dans de l'huile minérale, et vendu sous la dénomination Salcare SC95 par la société Allied Colloids, en ce qui concerne le "Polyquaternium 32", au copolymère réticulé de l'acrylamide et du chlorure de méthacryloyloxyéthyltriméthylammonium (20/80 en poids), en dispersion à 50% dans de l'huile minérale, et vendu sous la dénomination Salcare SC92 par la société Allied Colloids, et en ce qui concerne le "Polyquaternium 35", au méthosulfate du copolymère de méthacryloyloxyéthyltriméthylammonium et de méthacryloyloxyéthyldiméthylacétylammonium, vendu sous la dénomination Plex 7525L par la société Rohm GmbH.

Parmi les polymères substantifs cationiques ou amphotères utilisables selon l'invention, on préfère notamment mettre en oeuvre :

- l'homopolymère de chlorure de diméthyldiallylammonium vendu sous la dénomination "Merquat 100", par la société Merck ;

- les copolymères du chlorure de diméthyldiallylammonium et de l'acrylamide vendus sous les dénominations "Merquat 550" et "Merquat S" par la société Merck ;

- les polymères de polyammonium quaternaire préparés et décrits dans le brevet français 2 270 846, constitués de motifs récurrents répondant à la formule (II) suivante :

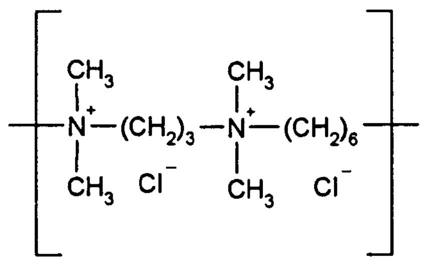 (II)

et dont le poids moléculaire, déterminé par chromatographie par perméation de gel, est compris entre 9500 et 9900 ;

- les polymères de polyammonium quaternaire préparés et décrits dans le brevet français 2 270 846, constitués de motifs récurrents répondant à la formule (III) suivante :

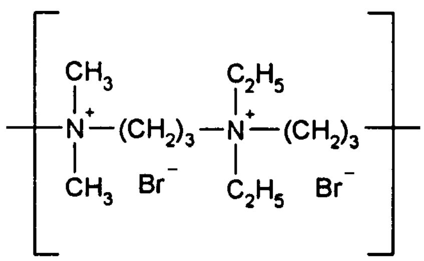 (III)

et dont le poids moléculaire, déterminé par chromatographie par perméation de gel, est de 1200 ;

- les polymères de polyammonium quaternaire, décrits et préparés dans les brevets US 4 157 388, 4 390 689, 4 702 906, 4 719 282, et constitués de motifs récurrents répondant à la formule (IV) suivante :

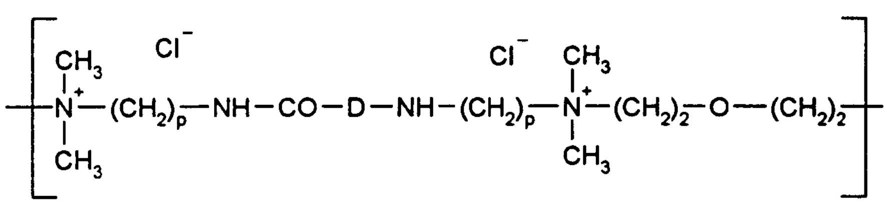 (IV)

dans laquelle p désigne un nombre entier variant de 1 à 6 environ, D peut être nul ou peut représenter un groupement $-(CH_2)_r-CO-$ dans lequel r désigne un nombre égal à 4 ou à 7, la masse moléculaire desdits polymères étant inférieure à 100 000, de préférence inférieure ou égale à 50 000 ; de tels polymères sont notamment vendus par la société Miranol sous les dénominations "Mirapol A15", "Mirapol AD1", "Mirapol AZ1" et "Mirapol 175";

- le copolymère de chlorure de diallyldiméthylammonium et d'acide acrylique (80/20) vendu sous la dénomination Merquat 280 par la société Calgon.

La concentration en polymère substantif cationique ou amphotère peut varier entre 0,02 et 10 % en poids environ par rapport au poids total de la composition de teinture appliquée sur les cheveux, et de préférence entre 0,05 et 5 % en poids.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie au moyen soit d'agents alcalinisants habituellement utilisés en teinture des fibres kératiniques, tels que l'ammoniaque, les carbonates alcalins, les alcanolamines, par exemple les mono-, di- et tri- éthanolamines ainsi que leurs dérivés, les hydroxydes de sodium ou de potassium, les composés de formule :

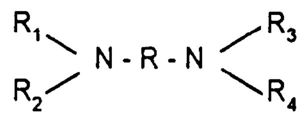

dans laquelle R est un reste propylène éventuellement substitué par un groupement hydroxyle ou un radical alkyle en $C_1$-$C_4$ ; $R_1$ $R_2$, $R_3$ et $R_4$, simultanément ou indépendamment l'un de l'autre, représentent un atome d'hydrogène, un radical alkyle en $C_1$-$C_4$ ou hydroxyalkyle en $C_1$-$C_4$,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus est tel qu'après mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Comme indiqué précédemment, les compositions de teinture préférées selon l'invention contiennent en outre, à côté des précurseurs de colorants d'oxydation définis ci-dessus, un ou plusieurs coupleurs.

Parmi ces coupleurs, on peut citer plus particulièrement : le 5-amino 2-méthyl phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole et le 4-hydroxy indole. On peut également mettre en oeuvre d'autres coupleurs usuels.

Les compositions de teinture peuvent encore contenir, en plus des précurseurs de colorants d'oxydation définis ci-dessus et des éventuels coupleurs associés, d'autres précurseurs de colorants d'oxydation ainsi que des colorants directs, notamment pour modifier les nuances ou les enrichir en reflets. Leur concentration pondérale peut varier entre 0,0005 et 10% environ, et de préférence entre 0,001 et 5% environ, par rapport au poids total de la composition de teinture appliquée sur les cheveux.

Parmi les précurseurs de colorants d'oxydation complémentaires utilisables dans le cadre de la présente invention, on peut citer de préférence : le 4-aminophénol, le 4-amino 2-méthyl phénol, le 4-amino 3-méthyl phénol, le 4-amino 2-hydroxyméthyl phénol, le 4-amino 3-hydroxyméthyl phénol, la paraphénylènediamine, la paratoluylènediamine, la 2-isopropyl paraphénylènediamine, la 2-(β-hydroxyéthyloxy) paraphénylènediamine, le 1-,N-bis-(β-hydroxyéthyl) amino 4-amino benzène et le 1-(β-méthoxyéthyl) amino 4-amino benzène.

Les colorants directs, quant à eux, peuvent être choisis parmi les colorants nitrés, azoïques ou anthraquinoniques.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tert.butyl hydroquinone et l'acide homogentisique, et ils sont alors généralement présents dans des proportions comprises entre environ 0,05 et 1,5 % en poids par rapport au poids total de la composition.

Les compositions de teinture selon l'invention contiennent également, dans leur forme de réalisation préférée, des agents tensioactifs bien connus de la technique, dans des proportions comprises entre environ 0,5 et 55 % en poids, et de préférence entre 2 et 50 % en poids par rapport au poids total de la composition, des solvants organiques, dans des proportions comprises entre environ 1 et 40 % en poids, et en particulier entre 5 et 30 % en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés.

Dans ces exemples, le critère de sélectivité de la teinture est évalué au moyen de l'indice de variation de couleur **I** calculé selon l'équation de NICKERSON suivante (voir à cet égard "Journal of the Optical Society of America", 1944, Sept., Vol 34, n° 9, p 550-570) :

$$I = 0{,}4\ Co\ \Delta H + 6\Delta V + 3\Delta C$$

dans laquelle les paramètres H,V, et C représentent les paramètres de la notation MUNSELL (Norme ASTM D 1535-68) qui définit la couleur (H désignant la nuance ou HUE, V désignant l'intensité ou VALUE, C la pureté ou CHROMATICITE et Co la pureté ou CHROMATICITE de la mèche par rapport à laquelle on désire évaluer la variation de la couleur).

## EXEMPLE 1

On a préparé la composition de teinture, conforme à l'invention, suivante :

| | |
|---|---|
| - 2-(β-hydroxyéthyl) paraphénylènediamine, dichlorhydrate | 0,675 g |
| - 5-N-(β-hydroxyéthyl)amino 2-méthyl phénol | 0,501 g |
| - Octyldodécanol vendu sous la dénomination EUTANOL G par la société Henkel | 8,0 g |
| - Acide oléique | 20,0 g |
| - Lauryléthersulfate de monoéthanolamine vendu sous la dénomination Sipon LM35 par la société Henkel | 3,0 g |
| - Alcool éthylique | 10,0 g |
| - Alcool benzylique | 10,0 g |
| - Alcool cétylstéarylique à 33 moles d'oxyde d'éthylène vendu sous la dénomination Simulsol GS par la société Seppic | 2,4 g |
| - Acide éthylènediaminetétracétique | 0,2 g |
| - Solution aqueuse à 60% de Matière Active (M.A.) du polymère de polyammonium quaternaire de formule (II) | 2,2 g M.A. |
| - Monoéthanolamine | 7,5 g |
| - Diéthanolamide d'acide linoléique vendu sous la dénomination Comperlan F par la société Henkel | 8,0 g |
| - Métabisulfite de sodium en solution aqueuse à 35% de M.A. | 0,46gM.A. |
| - Parfum, conservateur, | q.s. |
| - Ammoniaque à 20 % de $NH_3$ | 10,2 g |
| - Hydroquinone | 0,15 g |
| - 1-phényl 3-méthyl 5-pyrazolone | 0,2 g |
| - Eau déminéralisée q.s.p. | 100 g |

Au moment de l'emploi, on a mélangé cette composition poids pour poids avec une solution d'eau oxygénée titrant 20 volumes (6% en poids) et de pH 3.

On a obtenu un mélange de pH 9,8.

On a ensuite apprécié, et comparé, les critères de puissance et de sélectivité de teinture attachés à la composition obtenue, et ceci de la façon suivante :

Evaluation de la puissance :

On a appliqué le mélange ci-dessus sur des cheveux châtains moyennement décolorés, pendant 30 minutes. Après rinçage, lavage au shampooing, rinçage et séchage, les cheveux sont teints dans une nuance, qui, chiffrée en valeur MUNSELL sur un colorimètre MINOLTA CM 2002, correspond à la valeur suivante : 1,5 R 2,9/3,1.

A titre comparatif, on a appliqué sur ces mêmes cheveux châtains moyennement décolorés, et dans les mêmes conditions, un contretype du mélange ci-dessus qui ne renfermait pas de polymère de polyammonium quaternaire (le reste de la composition étant donc égal par ailleurs). La nuance alors obtenue correspond à la valeur suivante : 3,1 R 3,3/3,2.

Les cheveux châtains moyennement décolorés non teints (état initial) présentaient une nuance correspondant à la valeur suivante : 1,7 Y 6,2/4,6.

L'équation de NICKERSON, appliquée à ces valeurs, donne des indices de variation de couleur entre cheveux teints et non teints de :

**I =** 61,47 pour les cheveux teints au moyen de la composition conforme à l'invention

**I =** 55,82 pour les cheveux teints au moyen de la composition comparative, c'est à dire sans polymère de polyammonium quaternaire,

ce qui démontre une puissance de teinture plus importante avec la composition selon l'invention.

Evaluation de la sélectivité :

En appliquant le mélange conforme à l'invention préparé ci-dessus, et dans les mêmes conditions de traitement, mais cette fois sur des cheveux gris à 90% de blancs, on a obtenu une nuance MUNSELL de : 9,2 RP 3,9 / 2,5 , alors que par application du mélange contretype exempt de polymère de polyammonium quaternaire, on a obtenu une nuance MUNSELL de : 10 RP 4,0 / 2,5.

Toujours en appliquant le mélange conforme à l'invention préparé ci-dessus, et dans les mêmes conditions de traitement, mais cette fois sur des cheveux fortement décolorés, on a obtenu une nuance MUNSELL de : 9,2 RP 3,3 / 3,3 , alors que par application du mélange contretype exempt de polymère de polyammonium quaternaire, on a obtenu une nuance MUNSELL qui était de : 9,8 RP 3,3 / 3,3.

La variation de couleur exprimée par l'équation de NICKERSON entre cheveux 90% blancs et cheveux fortement décolorés est donc égale à 3,0 dans le cas du mélange préparé à partir de la composition conforme à l'invention (c'est à dire contenant le polymère de polyammonium quaternaire), alors que cette variation de couleur est égale à 5,9 dans le cas de la composition contretype (c'est à dire sans polymère).

Ces derniers résultats démontrent qu'une chevelure fortement décolorée et comportant 90% de cheveux blancs en racines, présente, après teinture au moyen de la composition selon l'invention (i.e. avec polymère substantif), une coloration bien plus uniforme qu'avec une composition de l'art antérieur (i.e. sans polymère substantif).

**EXEMPLE 2**

On a préparé la composition de teinture, conforme l'invention, suivante :

| | |
|---|---|
| - 2-(β-hydroxyéthyloxy) paraphénylènediamine, dichlorhydrate | 0,480 g |
| - 5-N-(β-hydroxyéthyl)amino 2-méthyl phénol | 0,33 g |
| - Octyldodécanol vendu sous la dénomination EUTANOL G par la société Henkel | 8,0 g |
| - Acide oléique | 20,0 g |
| - Lauryléthersulfate de monoéthanolamine vendu sous la dénomination Sipon LM35 par la société Henkel | 3,0 g |
| - Alcool éthylique | 10,0 g |
| - Alcool benzylique | 10,0 g |
| - Alcool cétylstéarylique à 33 moles d'oxyde d'éthylène vendu sous la dénomination Simulsol GS par la société Seppic | 2,4 g |
| - Acide éthylènediaminetétracétique | 0,2 g |
| - Polymère de polyammonium quaternaire vendu en solution aqueuse à 52% de M.A., par la société Miranol sous la dénomination Mirapol A15 | 2,08 g M.A. |
| - Monoéthanolamine | 7,5 g |
| - Diéthanolamide d'acide linoléique vendu sous la dénomination Comperlan F par la société Henkel | 8,0 g |
| - Métabisulfite de sodium en solution aqueuse à 35% de M.A. | 0,46 g M.A. |
| - Parfum, conservateur, | q.s. |
| - Ammoniaque à 20 % de $NH_3$ | 10,2 g |
| - Hydroquinone | 0,15 g |
| - 1-phényl 3-méthyl 5-pyrazolone | 0,2 g |
| - Eau déminéralisée q.s.p. | 100 g |

Au moment de l'emploi, on a mélangé cette composition poids pour poids avec une solution d'eau oxygénée titrant 20 volumes (6% en poids) et de pH 3.
On a obtenu un mélange de pH 9,8.
On a ensuite appliqué ce mélange sur des cheveux sensibilisés à des degrés divers, pendant 30 minutes. Après rinçage, lavage au shampooing, rinçage et séchage, les cheveux étaient teints dans une nuance puissante et peu sélective, c'est à dire présentant une bonne uniformité.

**EXEMPLE 3**

On a préparé la composition de teinture, conforme l'invention, suivante :

| | |
|---|---|
| - 2-(β-hydroxyéthyl) paraphénylènediamine, dichlorhydrate | 0,450 g |
| - 5-amino 2-méthyl phénol | 0,250 g |
| - Octyldodécanol vendu sous la dénomination EUTANOL G par la société Henkel | 8,0 g |
| - Acide oléique | 20,0 g |
| - Lauryléthersulfate de monoéthanolamine vendu sous la dénomination Sipon LM35 par la société Henkel | 3,0 g |
| - Alcool éthylique | 10,0 g |
| - Alcool benzylique | 10,0 g |
| - Alcool cétylstéarylique à 33 moles d'oxyde d'éthylène vendu sous la dénomination Simulsol GS par la société Seppic | 2,4 g |
| - Acide éthylènediaminetétracétique | 0,2 g |
| - Homopolymère de chlorure de diméthyldiallylammonium vendu en solution aqueuse à 40% de M.A., par la société Merck sous la dénomination Merquat 100 | 2,0 g M.A. |
| - Monoéthanolamine | 7,5 g |
| - Diéthanolamide d'acide linoléique vendu sous la dénomination Comperlan F par la société Henkel | 8,0 g |
| - Métabisulfite de sodium en solution aqueuse à 35% de M.A. | 0,46gM.A. |
| - Parfum, conservateur, | q.s. |
| - Ammoniaque à 20 % de $NH_3$ | 10,2 g |
| - Hydroquinone | 0,15 g |
| - 1-phényl 3-méthyl 5-pyrazolone | 0,2 g |
| - Eau déminéralisée q.s.p. | 100 g |

Au moment de l'emploi, on a mélangé cette composition poids pour poids avec une solution d'eau oxygénée titrant 20 volumes (6% en poids) et de pH 3.
On a obtenu un mélange de pH 9,8.
On a ensuite appliqué ce mélange sur des cheveux sensibilisés à des degrés divers, pendant 30 minutes. Après rinçage, lavage au shampooing, rinçage et séchage, les cheveux étaient teints dans une nuance puissante et peu sélective, c'est à dire présentant une bonne uniformité.

**EXEMPLE 4**

On a préparé la composition de teinture, conforme l'invention, suivante :

| | |
|---|---|
| - 2-(β-hydroxyéthyl) paraphénylènediamine, dichlorhydrate | 0,337 g |
| - 2,4-diamino 1-(β-hydroxyéthyloxy) benzène, dichlorhydrate | 0,361 g |
| - Alcool cétylstéarylique | 10,0 g |
| - Alcool oléocétylique oxyéthyléné à 30 moles d'oxyde d'éthylène | 5,0 g |
| - Lauryléthersulfate de sodium et de magnésium en solution à 26% de M.A. | 2,6 g M.A. |
| - Copolymère de chlorure de diméthyldiallylammonium et d'acide acrylique (80/20) en solution aqueuse à 35% de M.A., vendu par la société Merck sous la dénomination Merquat 280 | 1,75 g M.A. |
| - Métabisulfite de sodium en solution aqueuse à 35% de M.A. | 0,46 g M.A. |
| - Parfum, conservateur, | qs |
| - Acide diéthylènetriaminepentacétique à 40% de M.A. | 0,8 g M.A. |
| - Ammoniaque à 20 % de $NH_3$ | 10,2 g |
| - Hydroquinone | 0,15 g |
| - 1-phényl 3-méthyl 5-pyrazolone | 0,2 g |
| - Eau déminéralisée q.s.p. | 100 g |

Au moment de l'emploi, on a mélangé cette composition poids pour poids avec une solution d'eau oxygénée titrant 20 volumes (6% en poids) et de pH 3.
On a obtenu un mélange de pH 9,8.
On a ensuite appliqué ce mélange sur des cheveux sensibilisés à des degrés divers, pendant 30 minutes. Après rinçage, lavage au shampooing, rinçage et séchage, les cheveux étaient teints dans une nuance puissante et peu sélective, c'est à dire présentant une bonne uniformité.

## Revendications

1. Composition de teinture d'oxydation pour fibres kératiniques, en particulier pour fibres kératiniques humaines telles que les cheveux, du type comprenant dans un milieu approprié pour la teinture, au moins un précurseur de colorant d'oxydation et, le cas échéant, un ou plusieurs coupleurs, caractérisée par le fait qu'elle contient (i) à titre de précurseur de colorant d'oxydation, au moins une paraphénylènediamine choisie parmi les composés de formule (I) suivante :

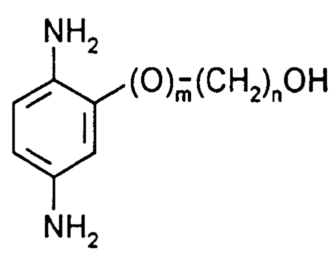

(I)

dans laquelle m est un nombre entier égal à zéro ou 1, et n est un nombre entier compris inclusivement entre 1 et 4, et les sels d'addition de ces composés de formule (I) avec un acide,
et (ii) au moins un polymère substantif cationique ou amphotère.

**2.** Composition selon la revendication 1, caractérisée par le fait que la paraphénylènediamine de formule (I) est choisie parmi la 2-(β-hydroxyéthyl) paraphénylènediamine, la 2-(β-hydroxyéthyloxy) paraphénylènediamine et leurs sels d'addition avec un acide.

**3.** Composition selon la revendication 1 ou 2, caractérisée par le fait que le polymère substantif cationique est un homopolymère de chlorure de diméthyldiallylammonium.

**4.** Composition selon la revendication 1 ou 2, caractérisée par le fait que le polymère substantif cationique est un copolymère de diméthyldiallylammonium et de l'acrylamide.

**5.** Composition selon la revendication 1 ou 2, caractérisée par le fait que le polymère substantif cationique est un polymère de polyammonium quaternaire constitué de motifs récurrents répondant la formule (II) suivante :

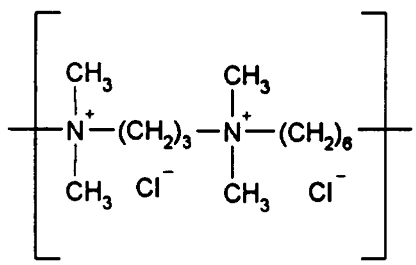

(II)

ledit polymère ayant un poids moléculaire, déterminé par chromatographie par perméation de gel, compris entre 9500 et 9900.

**6.** Composition selon la revendication 1 ou 2, caractérisée par le fait que le polymère substantif cationique est un polymère de polyammonium quaternaire constitué de motifs récurrents répondant la formule (III) suivante :

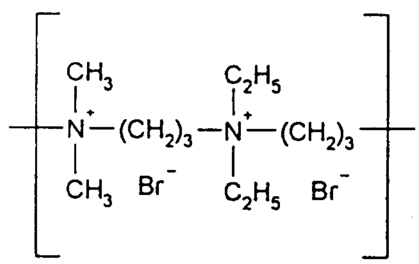

(III)

ledit polymère ayant un poids moléculaire, déterminé par chromatographie par perméation de gel, de 1200.

**7.** Composition selon la revendication 1 ou 2, caractérisée par le fait que le polymère substantif cationique est un polymère de polyammonium quaternaire constitué de motifs récurrents répondant la formule (IV) suivante :

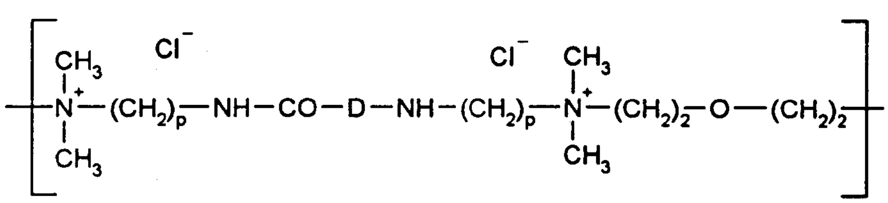

(IV)

dans laquelle p désigne un nombre entier variant de 1 à 6 environ, D peut être nul ou peut représenter un groupement $-(CH_2)_r-CO-$ dans lequel r désigne un nombre égal à 4 ou à 7, la masse moléculaire dudit polymère étant inférieure à 100 000, de préférence inférieure ou égale à 50 000.

8. Composition selon la revendication 1 ou 2, caractérisée par le fait que le polymère substantif amphotère est un copolymère de diallyldiméthylammonium et d'acide acrylique.

9. Composition selon l'une quelconque des revendications précédentes, caractérisée par le fait que les sels d'addition avec un acide de la paraphénylènediamine de formule (I) sont choisis parmi les chlorhydrates, les sulfates, les bromhydrates et les tartrates.

10. Composition selon l'une quelconque des revendications précédentes, caractérisée par le fait que la paraphénylènediamine de formule (I), ou ses sels, est présente dans une concentration comprise entre 0,05 et 10 % en poids par rapport au poids total de la composition, et de préférence entre 0,1 et 5 % en poids.

11. Composition selon l'une quelconque des revendications précédentes, caractérisée par le fait que le polymère substantif cationique ou amphotère est présent dans des concentrations comprises entre 0,02 et 10% en poids par rapport au poids total de la composition appliquée sur les cheveux.

12. Composition selon l'une quelconque des revendications précédentes, caractérisée par le fait qu'elle contient en outre un ou plusieurs coupleurs.

13. Composition selon l'une quelconque des revendications précédentes, caractérisée par le fait qu'elle contient en outre des colorants directs et/ou des précurseurs de colorants d'oxydation complémentaires autres que ceux répondant à la formule (I).

14. Composition selon l'une quelconque des revendications précédentes, prête à l'emploi, caractérisée par le fait qu'elle contient en outre un agent oxydant et qu'elle possède un pH compris entre 3 et 11.

15. Procédé de teinture des fibres kératiniques et en particulier des fibres kératiniques humaines telles que les cheveux, caractérisé par le fait qu'il consiste à appliquer sur les fibres une composition de teinture (A1) telle que définie à l'une quelconque des revendications 1 à 13, et à révéler la couleur en milieu alcalin neutre ou acide à l'aide d'un agent oxydant qui est ajouté juste au moment de l'emploi à cette composition (A1) ou qui est présent dans une composition (B1) appliquée simultanément ou séquentiellement de façon séparée.

16. Procédé de teinture des fibres kératiniques et en particulier des fibres kératiniques humaines telles que les cheveux, caractérisé par le fait qu'il consiste à appliquer sur les fibres une composition de teinture (A2) contenant, dans un milieu approprié pour la teinture, au moins une paraphénylènediamine de formule (I), telle que définie dans la revendication 1 éventuellement un ou plusieurs coupleurs, en la présence ou en l'absence d'un polymère substantif cationique ou amphotère, et à révéler la couleur en milieu alcalin, neutre ou acide à l'aide d'une composition oxydante (B2) contenant un agent oxydant et au moins un polymère substantif cationique ou amphotère, et qui est mélangée juste au moment de l'emploi à la composition (A2) ou qui est appliquée simultanément ou séquentiellement de façon séparée.

17. Dispositif à plusieurs compartiments ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A1) telle que définie à l'une quelconque des revendications 1 à 13, et un autre une composition (B1) comprenant un agent oxydant dans un milieu approprié pour la teinture.

18. Dispositif à plusieurs compartiments ou "kit" pour la teinture des fibres kératiniques et en particulier des fibres kératiniques humaines telles que les cheveux, caractérisé par le fait qu'il comporte au moins deux compartiments, dont l'un d'entre eux renferme une composition (A2) telle que définie dans la revendication 16, et un autre une composition (B2) telle que définie dans la revendication 16.

19. Utilisation d'une composition de teinture d'oxydation telle que définie à l'une quelconque des revendications 1 à 14 ou d'un dispositif de teinture ou "Kit" à plusieurs compartiments tel que défini à la revendication 17 ou 18 , pour la teinture des fibres kératiniques humaines telles que les cheveux.

## Claims

1. Oxidation dye composition for keratin fibres, in particular for human keratin fibres such as hair, of the type comprising, in a medium which is suitable for dyeing, at least one oxidation dye precursor and, if required, one or more couplers, which is characterized in that it contains (i) as oxidation dye precursor, at least one para-phenylenediamine

chosen from the compounds of formula (I) below:

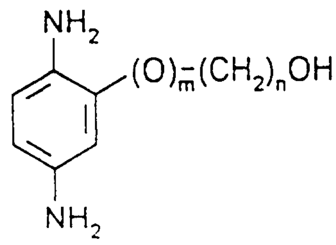

(I)

in which m is an integer equal to zero or 1 and n is an integer between 1 and 4 inclusively, and the addition salts of these compounds of formula (I) with an acid, and (ii) at least one cationic or amphoteric substantive polymer.

2. Composition according to Claim 1, characterized in that the para-phenylenediamine of formula (I) is chosen from 2-(β-hydroxyethyl)-para-phenylenediamine, 2-(β-hydroxyethyloxy)-para-phenylenediamine and the addition salts thereof with an acid.

3. Composition according to Claim 1 or 2, characterized in that the cationic substantive polymer is a dimethyldiallylammonium chloride homopolymer.

4. Composition according to Claim 1 or 2, characterized in that the cationic substantive polymer is a copolymer of dimethyldiallylammonium chloride and acrylamide.

5. Composition according to Claim 1 or 2, characterized in that the cationic substantive polymer is a quaternary polyammonium polymer consisting of repeating units corresponding to the following formula (II):

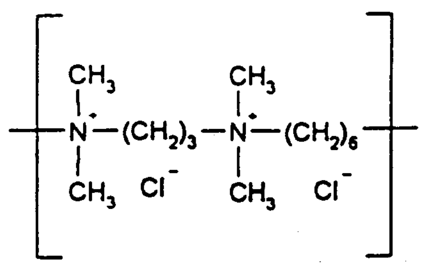

(II)

the said polymer having a molecular weight, determined by gel permeation chromatography, between 9500 and 9900.

6. Composition according to Claim 1 or 2, characterized in that the cationic substantive polymer is a quaternary polyammonium polymer consisting of repeating units corresponding to the following formula (III):

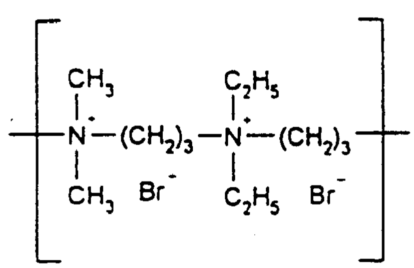

(III)

the said polymer having a molecular weight, determined by gel permeation chromatography, of 1200.

**7.** Composition according to Claim 1 or 2, characterized in that the cationic substantive polymer is a quaternary polyammonium polymer consisting of repeating units corresponding to the following formula (IV):

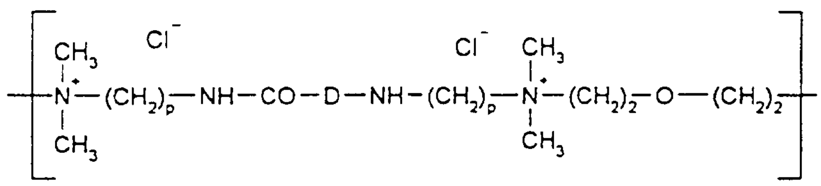 (IV)

in which p denotes an integer ranging approximately from 1 to 6, D may be nothing or may represent a group $-(CH_2)_r-CO-$ in which r denotes a number equal to 4 or to 7, the molecular mass of the said polymer being less than 100,000, preferably less than or equal to 50,000.

**8.** Composition according to Claim 1 or 2, characterized in that the amphoteric substantive polymer is a copolymer of diallyldimethylammonium chloride and acrylic acid.

**9.** Composition according to any one of the preceding claims, characterized in that the addition salts with an acid for the para-phenylenediamine of formula (I) are chosen from the hydrochlorides, the sulphates, the hydrobromides and the tartrates.

**10.** Composition according to any one of the preceding claims, characterized in that the para-phenylenediamine of formula (I), or the salts thereof, is present in a concentration between 0.05 and 10 % by weight, relative to the total weight of the composition, and preferably between 0.1 and 5 % by weight.

**11.** Composition according to any one of the preceding claims, characterized in that the cationic or amphoteric substantive polymer is present in concentrations between 0.02 and 10 % by weight relative to the total weight of the composition applied to the hair.

**12.** Composition according to any one of the preceding claims, characterized in that it additionally contains one or more couplers.

**13.** Composition according to any one of the preceding claims, characterized in that it additionally contains direct dyes and/or additional oxidation dye precursors other than those corresponding to the formula (I).

**14.** Composition according to any one of the preceding claims, which is ready to use, characterized in that it additionally contains an oxidizing agent and in that it has a pH between 3 and 11.

**15.** Process for dyeing keratin fibres and in particular human keratin fibres such as hair, characterized in that it consists in applying to the fibres a dye composition (A1) as defined in any one of Claims 1 to 13, and in developing the colour in an alkaline, neutral or acidic medium using an oxidizing agent which is added to this composition (A1) only at the time of use or which is present in a composition (B1) that is applied simultaneously or sequentially in a separate manner.

**16.** Process for dyeing keratin fibres and in particular human keratin fibres such as hair, characterized in that it consists in applying to the fibres a dye composition (A2) containing, in a medium which is suitable for dyeing, at least one para-phenylenediamine of formula (I) as defined in Claim 1 and one or more optional couplers, in the presence or absence of a cationic or amphoteric substantive polymer, and in developing the colour in an alkaline, neutral or acidic medium using an oxidizing composition (B2) containing an oxidizing agent and at least one cationic or amphoteric substantive polymer, and which is mixed with the composition (A2) only at the time of use or which is applied simultaneously or sequentially in a separate manner.

**17.** Multi-compartment device or "kit" for dyeing keratin fibres and in particular human keratin fibres such as hair, characterized in that it contains at least two compartments, one of which contains a composition (A1) as defined in any one of Claims 1 to 13, and another of which contains a composition (B1) comprising an oxidizing agent in a medium which is suitable for dyeing.

**18.** Multi-compartment device or "kit" for dyeing keratin fibres and in particular human keratin fibres such as hair, characterized in that it contains at least two compartments, one of which contains a composition (A2) as defined in Claim 16, and another of which contains a composition (B2) as defined in Claim 16.

**19.** Use of an oxidation dye composition as defined in any one of Claims 1 to 14 or of a multicompartment dyeing device or "kit" as defined in Claim 17 or 18, for dyeing human keratin fibres such as hair.

**Patentansprüche**

**1.** Zusammensetzung zum oxidativen Färben von Keratinfasern, insbesondere von menschlichen Keratinfasern, wie dem Haar, die in einem zum Färben geeigneten Medium mindestens ein Farbstoffvorprodukt einer Oxidationsfarbe und gegebenenfalls ein oder mehrere Kuppler enthält, dadurch gekennzeichnet, daß sie (i) als Farbstoffvorprodukt der Oxidationsfarbe mindestens ein p-Phenylendiamin enthält, das unter den folgenden Verbindungen der Formel (I) ausgewählt ist:

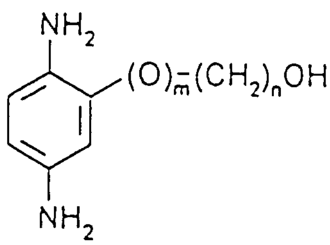

(I)

worin bedeuten: m Null oder eins und n eine ganze Zahl im Bereich von 1 bis 4,
und die Additionssalze dieser Verbindungen der Formel (I) mit einer Säure,
und (ii) mindestens ein im substantives kationisches oder amphoteres Polymer enthält.

**2.** Zusammensetzung nach Anspruch 1, dadurch gekennzeichnet, daß das p-Phenylendiamin der Formel (I) unter 2-(β-Hydroxyethyl)-p-phenylendiamin, 2-(β-Hydroxyethyloxy)-p-phenylendiamin und den Additionssalzen dieser Verbindungen mit einer Säure ausgewählt ist.

**3.** Zusammensetzung nach Anspruch 1 oder 2, dadurch gekennzeichnet, daß das substantive kationische Polymer ein Homopolymer von Dimethyldiallylammoniumchlorid ist.

**4.** Zusammensetzung nach Anspruch 1 oder 2, dadurch gekennzeichnet, daß das substantive kationische polymer ein Copolymer aus Dimethyldiallylammonium und Acrylamid ist.

**5.** Zusammensetzung nach Anspruch 1 oder 2, dadurch gekennzeichnet, daß das substantive kationische Polymer ein Polymer mit mehreren quaternären Ammoniumgruppen ist, das aus sich wiederholenden Einheiten der nachfolgenden Formel (II) besteht:

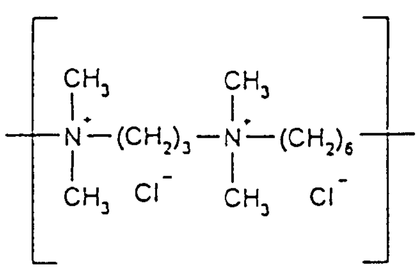

(II)

wobei das Polymer ein mit Gel-Permeations-Chromatographie bestimmtes Molekülgewicht im Bereich von 9500 bis 9900 aufweist.

6. Zusammensetzung nach Anspruch 1 oder 2, dadurch gekennzeichnet, daß das substantive kationische Polymer ein Polymer mit mehreren quaternären Ammoniumgruppen ist, das aus wiederkehrenden Einheiten der folgenden Formel (III) besteht:

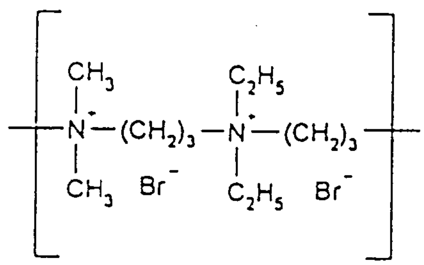

(III)

wobei das Polymer ein mit Gel-Permeations-Chromatographie bestimmtes Molekülgewicht von 1200 aufweist.

7. Zusammensetzung nach Anspruch 1 oder 2, dadurch gekennzeichnet, daß das substantive kationische Polymer ein Polymer mit mehreren quaternären Ammoniumgruppen ist, das aus wiederkehrenden Einheiten der nachfolgenden Formel (IV) besteht:

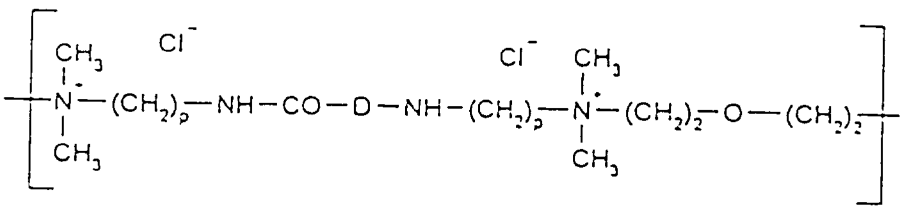

(IV)

worin p eine ganze Zahl im Bereich von etwa 1 bis 6 bedeutet; D kann fehlen oder eine Gruppe $-CH_2)_r-CO-$ bedeuten, worin r 4 oder 7 ist,
wobei das Polymer ein Molekülgewicht von unter 100 000 und vorzugsweise von unter oder gleich 50 000 aufweist.

8. Zusammensetzung nach Anspruch 1 oder 2, dadurch gekennzeichnet, daß das substantive amphotere Polymer ein Copolymer aus Diallyldimethylammonium und Acrylsäure ist.

9. Zusammensetzung nach einem der vorhergehenden Ansprüche, dadurch gekennzeichnet, daß das Additionssalz mit einer Säure des p-Phenylendiamins der Formel (I) unter Hydrochloriden, Sulfaten, Hydrobromiden und Tartraten ausgewählt ist.

10. Zusammensetzung nach einem der vorhergehenden Ansprüche, dadurch gekennzeichnet, daß das p-Phenylendiamin der Formel (I) oder seine Salze in einer Konzentration von 0,05 bis 10 Gew.-%, bezogen auf das Gesamtgewicht der Zusammensetzung, und vorzugsweise von 0,1 bis 5 Gew.-% vorliegen.

11. Zusammensetzung nach einem der vorhergehenden Ansprüche, dadurch gekennzeichnet, daß das substantive kationische oder amphotere Polymer in Konzentrationen von 0,02 bis 10 Gew.-%, bezogen auf das Gesamtgewicht der auf die Haare aufgetragenen Zusammensetzung vorliegt.

12. Zusammensetzung nach einem der vorhergehenden Ansprüche, dadurch gekennzeichnet, daß sie ferner einen oder mehrere Kuppler enthält.

13. Zusammensetzung nach einem der vorhergehenden Ansprüche, dadurch gekennzeichnet, daß sie ferner Direktfarbstoffe und/oder weitere Farbstoffvorprodukte von Oxidationsfarbstoffen enthält, die von den Farbstoffvorprodukten der Oxidationsfarben der Formel (I) verschieden sind.

14. Zusammensetzung nach einem der vorhergehenden Ansprüche, die gebrauchsfertig ist und die dadurch gekennzeichnet ist, daß sie ferner ein Oxidationsmittel enthält und daß sie einen pH-wert im Bereich von 3 bis 11 aufweist.

15. Verfahren zum Färben von Keratinfasern und insbesondere von menschlichen Keratinfasern, wie dem Haar, dadurch gekennzeichnet, daß es darin besteht, auf die Fasern eine Färbemittelzusammensetzung (A1) nach einem der Ansprüche 1 bis 13 aufzutragen, die Farbe in einem alkalischen, neutralen oder sauren Medium mit einem Oxidationsmittel zu entwickeln, das im Augenblick der Anwendung der Zusammensetzung (A1) zugesetzt wird oder das in einer Zusammensetzung (B1) vorliegt, die gleichzeitig oder daran anschließend aufgetragen wird.

16. Verfahren zum Färben von Keratinfasern und insbesondere menschlichen Keratinfasern, wie dem Haar, dadurch gekennzeichnet, daß es darin besteht, auf die Fasern eine Färbemittelzusammensetzung (A2) aufzutragen, die in einem zum Färben geeigneten Medium mindestens ein p-Phenylendiamin der Formel (I), nach Anspruch 1 und gegebenenfalls einen oder mehrere Kuppler in Gegenwart von oder ohne ein substantives kationisches oder amphoteres Polymer enthält, die Farbe in einem alkalischen, neutralen oder sauren Medium mit einer oxidierenden Zusammensetzung (B2) zu entwickeln, die ein Oxidationsmittel und mindestens ein substantives kationisches oder amphoteres Polymer enthält, und die im Augenblick der Anwendung mit der Zusammensetzung (A2) vermischt wird, oder sie wird gleichzeitig oder daran anschließend aufgetragen.

17. Vorrichtung mit mehreren Abteilungen oder "Kit" zum Färben von Keratinfasern und insbesondere menschlichen Keratinfasern, wie dem Haar, dadurch gekennzeichnet, daß sie mindestens zwei Abteilungen enthält, wobei eine der Abteilungen eine Zusammensetzung (A1) nach einem der Ansprüche 1 bis 13 und die andere Abteilung eine Zusammensetzung (B1), die das Oxidationsmittel in einem zum Färben geeigneten Medium enthält, enthalten.

18. Vorrichtung mit mehreren Abteilungen oder "Kits" zum Färben von Keratinfasern und insbesondere von menschlichen Keratinfasern, wie dem Haar, dadurch gekennzeichnet, daß es mindestens zwei Abteilungen enthält, wobei eine der Abteilungen eine Zusammensetzung (A2) nach Anspruch 16 und eine weitere Abteilung eine Zusammensetzung (B2) nach Anspruch 16 enthält.

19. Verwendung einer Zusammensetzung zum oxidativen Färben nach einem der Ansprüche 1 bis 14 oder einer Vorrichtung zum Färben oder eines "Kits" mit mehreren Abteilungen nach Anspruch 17 oder 18 zum Färben von menschlichen Keratinfasern, wie dem Haar.